Europäisches Patentamt

European Patent Office

Office européen des brevets

(19)

(11) Numéro de publication : **0 686 402 A1**

# DEMANDE DE BREVET EUROPEEN

(12)

(21) Numéro de dépôt : **95401185.4**

(22) Date de dépôt : **22.05.95**

(51) Int. Cl.⁶ : **A61L 31/00**

(30) Priorité : **08.06.94 FR 9406995**

(43) Date de publication de la demande :
**13.12.95 Bulletin 95/50**

(84) Etats contractants désignés :
**DE ES FR GB IT NL SE**

(71) Demandeur : **COLETICA**
**32, rue Saint Jean de Dieu**
**F-69007 Lyon (FR)**

(72) Inventeur : **Khoury, Wassim**
**66 rue de Ponthieu**
**F-75008 Paris (FR)**
Inventeur : **Abdul-Malak, Nabil**
**27 rue Frédéric Mistral**
**F-69300 Caluire (FR)**
Inventeur : **Huc, Alain**
**26 Chemin des Santons**
**F-69110 Ste Foy lès Lyon (FR)**

(74) Mandataire : **Portal, Gérard**
**Cabinet Beau de Loménie**
**158, rue de l'Université**
**F-75340 Paris Cédex 07 (FR)**

(54) **Membrane collagénique anti-adhérence post-opératoire**

(57)     L'invention concerne une membrane à base de collagène. Cette membrane est caractérisée en ce qu'elle comprend un support à base de collagène revêtu complètement d'une couche de gélatine, la couche de gélatine étant essentiellement non mélangée au support de collagène, de préférence l'ensemble de la couche de gélatine et du support à base de collagène étant à l'état lyophilisé.

L'invention trouve une application comme biomatériau en particulier en chirurgie notamment sous forme de pansements anti-adhérences.

EP 0 686 402 A1

La présente invention concerne essentiellement une membrane collagénique anti-adhérence post-opératoire.

Il est connu par le document FR-B1-2 628 634 un patch de chirurgie viscérale réalisé à partir d'un biomatériau formé de deux couches de collagène superposées et associées intimement, une première couche poreuse collable de collagène fibreux dans laquelle a pénétré au moins partiellement une solution de collagène destinée à former un film de collagène intimement lié à la première couche par pénétration profondément à l'intérieur des fibres de la première couche.

Dans une variante de réalisation, le collagène peut être remplacé au moins partiellement par de la gélatine (voir les revendications et page 3, lignes 6 à 30 et page 4, lignes 22 à 24). Il est indiqué en page 2, lignes 1 à 34, que ce patch permet une bonne cicatrisation des viscères, qu'il se colle facilement, réalise un excellent effet de confinement, d'hémostase en remplaçant temporairement la paroi reconstituée et qu'il résiste mécaniquement aussi bien sous forme sèche qu'après réhydratation dans un fluide physiologique tandis qu'il est également exempt de toxicité chimique tout en étant encore facilement colonisable par des cellules de l'organisme.

Cependant, il est apparu que le patch décrit dans ce document présente une résistance mécanique relativement faible car le film de collagène ou de gélatine est obtenu par collage d'une solution sur du collagène fibreux, ce qui entraîne une redissolution partielle de ce collagène fibreux et de ce fait la solution de collagène ou de gélatine se mélange en grande partie aux fibres collagéniques, but qui est recherché pour obtenir une imprégnation au moins partielle de la solution de collagène ou de gélatine profondément dans le collagène fibreux.

Egalement, les présents inventeurs ont découvert que ceci conduisait à divers inconvénients tels qu'une résistance mécanique relativement réduite, une faible adhésivité du patch aux organes pouvant conduire dans certains cas à une perte de contact intime avec l'organe, ainsi que des propriétés hémostatiques encore insuffisantes. En outre, les présents inventeurs ont également constaté que le patch décrit dans ce brevet est réalisé à partir d'atélocollagène, en étant obtenu par un procédé décrit dans les documents FR-A- 2 597 499 et FR-A-2 586 703 (voir page 4, lignes 6 à 10 et exemples 1 et 2). Or, l'atélocollagène est le produit de la digestion par la pepsine du collagène comme cela est clairement décrit dans le document FR-A-2 597 499 et il en résulte que le collagène fibreux obtenu est très soluble et qu'il va en conséquence se mélanger d'autant plus facilement à la solution de collagène ou de gélatine, mélange qui est par ailleurs un but essentiel de ce brevet et qui est donc recherché. Il en résulte une destruction partielle de la couche centrale de collagène fibreux. Au total, le produit final aura un effet barrière limité.

Par ailleurs, ce document est silencieux vis-à-vis du problème de la prévention des adhérences, ce qui fait que l'homme de l'art ne pouvait pas penser que ce produit pouvait être utilisé pour prévenir les adhérences.

Or, les adhérences post-opératoires sont responsables de complications plus ou moins graves.

En chirurgie abdominale, les adhérences intrapéritonéales multiplient par quatre le risque d'occlusion intestinale et peuvent provoquer des occlusions intestinales sur brides.

En gynécologie, les adhérences peuvent être la cause de stérilité et sont parfois à l'origine de douleurs pelviennes voire d'occlusions intestinales. Leur incidence est proportionnelle au nombre de réintervention.

Les adhérences péricardiques post-opératoires en chirurgie cardiaque constituent un problème majeur augmentant la mortalité lors des réinterventions. La sternotomie itérative comporte un risque potentiel des lésions du coeur et des gros vaisseaux ou des greffons extra-cardiaques dans 2 à 6 % des cas environ et en cas d'hémorragie importante lors de la resternotomie, la mortalité opératoire augmente considérablement de 37 à 50 %.

Puisque l'incidence de réintervention en chirurgie est en augmentation surtout chez les coronariens et que les adhérences sont également une cause majeure de complications dans les autres chirurgies, il est important d'améliorer la prévention des adhérences post-opératoires.

En effet, les nombreux travaux effectués dans ce domaine, principalement en chirurgie péricardique et péritonéale, ont eu des succès limités.

Les expériences réalisées tentaient d'agir sur les différentes étapes de la formation des adhérences.

Trois types d'essais ont été tentés :

- des traitements pharmacologiques par voie générale avec des corticoïdes à l'aide par exemple des médicaments contenant comme principe actif le vérapamil ou l'hydrocortisone. Les inconvénients de ces méthodes résident dans l'augmentation du risque infectieux, le retard de cicatrisation, la perturbation de l'hémostase et l'hypertension artérielle,
- l'irrigation-drainage réalisée avec des solutés type Dextran 70® de la société CLIN-MIDY. Cette technique pour être efficace nécessite un long séjour en réanimation avec un risque infectieux dû aux manipulations de nombreux flacons ajouté à un risque de tamponnade par mauvais drainage,
- le patch de Goretex® fabriqué par la société Gore : ce patch en polytétrafluoroéthylène a une efficacité très limité et il comporte des risques infectieux, de compression et de formation d'hématome.

La présente invention a pour but de résoudre le nouveau problème technique consistant en la réalisation d'un biomatériau permettant de réduire de façon très importante les risques d'apparition d'adhérence entre le biomatériau et les tissus des organes concernés par les opérations chirurgicales.

La présente invention a encore pour but de résoudre le nouveau problème technique énoncé ci-dessus en fournissant un biomatériau répondant simultanément aux critères suivants :
- capacité de prévention des adhérences entre le biomatériau et les organes concernés par les opérations chirurgicales,
- des propriétés mécaniques améliorées avec de préférence une très bonne résistance à la traction,
- biodégradabilité lente mais totale,
- la plus faible irritation possible des tissus avec lesquels il est en contact et aucune toxicité pour l'organisme,
- un pouvoir adhésif amélioré permettant d'améliorer la sécurité de maintien en contact intime avec l'organe concerné en renforçant ainsi les propriétés hémostatiques, ce qui est particulièrement important dans le cas où les organes opérés ne sont pas plans, ce qui est généralement le cas, ceci étant particulièrement vrai pour le foie qui présente une surface hépatique concave,
- structure assez compacte afin de jouer un rôle barrière,
- l'emploi d'un procédé de fabrication simple, de préférence ne nécessitant qu'une seule lyophilisation et si possible utilisant du collagène à l'état natif et obtenu par un procédé d'extraction simplifié,
- utilisation facile, et
- un coût raisonnable.

L'invention permet de résoudre ce nouveau problème technique tel qu'énoncé ci-dessus, complètement, d'une manière fiable et reproductible, utilisable à l'échelle industrielle et médicale, à un coût raisonnable, notamment grâce à un procédé de fabrication simple, de préférence ne nécessitant qu'une seule lyophilisation, permettant l'emploi de collagène à l'état natif et une procédure d'extraction simplifiée.

Dans le cadre de l'invention, il a été découvert que lorsqu'on dépose une couche distincte de gélatine sur au moins une face d'un support de collagène, on résout les problèmes techniques ci-dessus et on obtient l'avantage déterminant que la gélatine réalise un effet barrière de par sa structure compacte ainsi qu'un pouvoir collant élevé permettant un contact intime, à condition qu'elle forme une couche distincte de collagène, tout en obtenant des propriétés mécaniques élevées grâce au fait que le support de collagène n'est pas modifié.

Ainsi, selon un premier aspect, la présente invention fournit une membrane à base de collagène, caractérisée en ce qu'elle comprend un support à base dudit collagène revêtu complètement sur au moins une face d'une couche de gélatine, la couche de gélatine étant essentiellement non mélangée au support de collagène, de préférence l'ensemble de la couche de gélatine et du support à base de collagène étant à l'état lyophilisé.

Selon un mode de réalisation avantageux, la membrane selon l'invention est caractérisée en ce qu'elle se présente sous forme compacte, en particulier en ayant été comprimée à chaud.

Selon un autre mode de réalisation avantageux de l'invention, la membrane précitée est obtenue en déposant une solution de gélatine sur au moins une face d'un gel de collagène préalablement traité pour être non fluide et non miscible à une solution, de préférence par congélation, avant lyophilisation de l'ensemble, ce qui permet d'éviter que la gélatine ne se mélange au collagène et procure l'avantage déterminant que la gélatine reste à la surface du collagène pour revêtir complètement la surface du support de collagène tout en formant lors de la lyophilisation ultérieure de l'ensemble une couche de revêtement distincte adhérente avec le collagène. Il est à noter que cette couche de revêtement de gélatine est distincte, essentiellement non mélangée au support de collagène, par le fait qu'elle est coulée sur au moins une face du support de collagène à l'état congelé, ce qui empêche tout mélange, la gélatine lorsqu'elle est coulée sur le collagène congelé se congelant elle-même au moins à l'interface, puis l'ensemble de la couche de gélatine et de support à base de collagène étant séché dans des conditions empêchant tout mélange, les conditions préférées étant une lyophilisation de l'ensemble.

Grâce à l'invention, on résout les problèmes techniques précédemment énoncés avec l'ensemble des avantages techniques associés. En outre, on notera que la présence de gélatine en couche distincte recouvrant le collagène sur au moins une face va conférer à la membrane des propriétés hémostatiques particulièrement améliorées grâce à une amélioration du pouvoir adhérent de la gélatine aux organes concernés par les opérations chirurgicales en appliquant la couche de gélatine contre l'organe, ce qui permet aussi de bénéficier du pouvoir adhésif très important de la gélatine. L'invention permet d'éviter la pousse d'adhérence entre la membrane et les organes concernés par les opérations chirurgicales, ce qui constitue un progrès technique déterminant en raison du fait que les adhérences post-opératoires sont responsables de complications plus ou moins graves augmentant le taux de mortalité comme rappelé dans l'introduction de la présente description.

Selon un autre mode de réalisation avantageux, la membrane selon l'invention est obtenue en préparant tout d'abord une éponge de collagène par lyophilisation d'un gel de collagène, ensuite une étape de réticulation du collagène réalisée par un agent réticulant qui peut être, par exemple, choisi parmi le diphénylphosphorylazide (DPPA) en obtenant ainsi après lyophilisation et réticulation, une éponge réticulée qui est réhydratée puis congelée pour présenter une surface apparente essentiellement étanche, avant de couler une solution de gélatine sur au moins une face de ladite éponge réticulée, réhydratée et à l'état congelé, en quantité suffisante pour couvrir complètement le support de collagène sur au moins une face d'une couche de revêtement continu de gélatine. L'ensemble obtenu est ensuite séché, de préférence en étant lyophilisé.

Le lyophilisat formant une membrane ensuite obtenu peut être comprimé à chaud pour obtenir une membrane comprimée selon l'invention qui constitue ainsi une membrane préférée selon l'invention anti-adhérence et présentant deux couches distinctes, une couche externe formant film de revêtement de gélatine, sur au moins une face, essentiellement non mélangé au support formant couche interne en collagène.

Selon une variante de réalisation particulière, le collagène choisi est essentiellement de type I et de préférence provient de la peau de veau.

En effet, le collagène essentiellement de type I constitue le collagène préféré puisqu'il peut être extrait sous forme complètement native, c'est-à-dire avec sa structure hélicoïdale et ses télopeptides préservés. Dans le cadre de l'invention, il a été découvert que le caractère natif du collagène est important pour une application thérapeutique, notamment sous forme de pansement évitant les adhérences en chirurgie post-opératoire. Le collagène natif essentiellement de type I permet d'obtenir un biomatériau avec de meilleures propriétés mécaniques et une susceptibilité plus faible vis-à-vis de la digestion enzymatique. Dans le cadre de l'invention, on entend par collagène natif du collagène comportant ses télopeptides et sa structure hélicoïdale.

Dans ce cadre, il est préféré dans le cadre de l'invention que le support à base de collagène essentiellement de type I se présente sous forme d'une nappe obtenue par lyophilisation d'un gel de collagène essentiellement de type I, à l'état natif.

En outre, selon un mode de réalisation avantageux, il peut être réalisé une réticulation physique du support de collagène en particulier par séchage sous vide à une température au moins égale à 80°C, et de préférence au moins égale à 100°C, ce qui permet d'améliorer la résistance mécanique de la membrane finale et de diminuer sa vitesse de digestion.

On peut encore augmenter la compacité dudit matériau et diminuer sa vitesse de biodégradation en comprimant à chaud la membrane obtenue après lyophilisation, sous une pression élevée, par exemple supérieure à 100 kg/cm$^2$ et en particulier de l'ordre de 200 kg/cm$^2$.

Il est à noter que le collagène seul ne permet pas de résoudre de manière satisfaisante le problème de l'adhésivité.

Dans le cadre de l'invention, il a été découvert de manière inattendue qu'une couche de gélatine formant une couche distincte sur au moins une face d'un support de collagène permettait de résoudre ce problème en améliorant l'adhérence du biomatériau vis-à-vis de l'organe qui doit être recouvert pour l'isoler des tissus avoisinants.

On comprend ainsi que l'invention permet bien de résoudre le problème technique précédemment énoncé en totalité, de manière fiable, reproductible, utilisable à l'échelle industrielle et médicale.

Selon un deuxième aspect, la présente invention fournit également un procédé de fabrication d'une membrane à base de collagène, caractérisé en ce qu'il comprend les étapes essentielles suivantes :

a) on prépare tout d'abord un gel de collagène, de préférence un collagène de type I ;

b) on traite le gel de collagène dans des conditions rendant le collagène non fluide et non capable d'être miscible à une solution ;

c) on prépare une solution de gélatine et on coule cette solution de gélatine sur au moins une face du collagène traité pour être non miscible avec ladite solution de gélatine ; et

d) on sèche l'ensemble formé par la couche de gélatine recouvrant complètement le collagène sur au moins une face, de préférence par lyophilisation, en obtenant ainsi ladite membrane.

Selon un mode de réalisation particulier, après séchage, de préférence par lyophilisation, on peut réaliser une réticulation physique de la membrane, en particulier en soumettant la membrane à un traitement sous pression réduite à la chaleur, en particulier à une température supérieure à 80°C et mieux supérieure à 100°C. Avantageusement, cette pression réduite peut être inférieure à 1 mbar et de préférence inférieure à 0,5 mbar. Cependant, cette réticulation physique de la membrane ne semble pas à l'heure actuelle présenter d'avantages particuliers car elle conduit à une réticulation de la gélatine ce qui diminue ses propriétés d'anti-adhérence.

Selon un autre mode de réalisation avantageux du procédé selon l'invention, le traitement du gel de collagène pour le rendre non fluide et non miscible comprend une étape de congélation.

Selon un autre mode de réalisation, ce traitement du gel de collagène comprend une lyophilisation. La lyophilisation du gel de collagène est particulièrement intéressante lorsqu'on souhaite réaliser une réticulation physique du collagène, surtout lorsque celui-ci est du collagène natif comme préféré selon l'invention. L'étape de réticulation physique de la membrane peut être réalisée dans les conditions précédemment énoncées, à savoir en soumettant le collagène lyophilisé à un traitement sous pression réduite à la chaleur, en particulier à une température supérieure à 80°C et mieux supérieure à 100°C. Cette pression réduite peut également être inférieure à 1 mbar et de préférence inférieure à 0,5 mbar.

Selon un autre mode de réalisation avantageux, le procédé selon l'invention comprend tout d'abord la préparation d'une éponge de collagène par lyophilisation d'un gel de collagène, ensuite d'une étape de réticulation du collagène réalisée par un agent réticulant qui peut être, par exemple, choisi par le diphénylphosphorylazide (DPPA) en obtenant ainsi après lyophilisation et réticulation, une éponge réticulée qui est ensuite réhydratée puis congelée pour présenter une surface apparente essentiellement étanche, avant de couler une solution de gélatine sur au moins une face de ladite éponge réticulée, réhydratée et à l'état congelé, en quantité suffisante pour couvrir complètement sur au moins une face le support de collagène d'une couche de revêtement continu de gélatine. L'ensemble obtenu est ensuite séché, de préférence en étant lyophilisé.

Selon un autre mode de réalisation avantageux du procédé de l'invention, la membrane obtenue après lyophilisation de l'ensemble de la solution de gélatine et du collagène est compactée, en particulier compactée à chaud, à une pression avantageusement au moins égale à 100 kg/cm$^2$, en particulier d'environ 200 kg/cm$^2$.

Ainsi, grâce à la méthode de fabrication qui vient d'être décrite, il est possible de préparer selon une technique industrielle aisée à mettre en oeuvre, une membrane selon l'invention qui est souple, lentement biodégradable, aisément manipulable, et qui évite les adhérences post-opératoires.

Dans les cas où la membrane sera utilisée comme biomatériau, en particulier en chirurgie, dans des points de l'organisme où la dégradation enzymatique sera importante, il est préféré d'utiliser une membrane préparée à partir d'un gel de collagène qui aura été tout d'abord lyophilisé pour former une éponge, qui est ensuite soumise à un traitement de réticulation ou de tannage, en particulier par le diphénylphosphorylazide avant d'être enduite ou recouverte de la solution de gélatine, sur au moins une face, après réhydratation et congélation, puis l'ensemble sera à nouveau lyophilisé.

Le lyophilisat obtenu pourra ensuite être comprimé à chaud pour obtenir la membrane sous sa forme finale directement utilisable comme bio-matériau de chirurgie ayant des propriétés anti-adhérences remarquables permettant ainsi de constituer des pansements anti-adhérences.

Ainsi, selon un troisième aspect, la présente invention couvre également l'utilisation de la membrane précitée comme biomatériau, en particulier en chirurgie et notamment sous forme de pansement notamment à action anti-adhérence.

L'invention couvre également des pansements notamment à action anti-adhérence caractérisés en ce qu'ils comprennent au moins une membrane telle que précédemment définie.

Dans ce cadre, l'invention trouve une application particulière en chirurgie abdominale en évitant les adhérences intrapéritonéales qui multiplient par quatre le risque d'occlusion intestinale et qui peuvent provoquer des occlusions intestinales sur brides ; en encore en gynécologie où les adhérences peuvent être la cause de stérilité, ainsi que de douleurs pelviennes voire d'occlusions intestinales ; ou encore en chirurgie cardiaque ou les adhérences péricardiques post-opératoires constituent un problème majeur augmentant la mortalité.

Dans le cadre de l'invention pour l'un quelconque des aspect précédents, on observera que la proportion de collagène dans la solution de collagène de départ pour former le support de collagène peut varier dans les limites habituelles de proportion, en général entre 0,3 et 2 % en poids, une proportion actuellement préférée étant d'environ 0,75 % en poids de collagène dans la solution de collagène.

En ce qui concerne la solution de gélatine destinée à former la couche de revêtement continue sur au moins une face du support de collagène, la proportion de gélatine sera de préférence comprise entre 0,5 et 4 % en poids, une proportion préférée étant de l'ordre de 1,5 %.

Par ailleurs, l'épaisseur de la couche de la solution de gélatine destinée à former le revêtement continu sur au moins une face du support de collagène n'est pas critique dès lors qu'elle est continue. Généralement, l'épaisseur de la couche pourrait être comprise entre 0,1 et 5 mm, exceptionnellement atteindre 10 mm. Naturellement, on peut déposer une couche de gélatine sur deux faces opposées du support ou sur l'ensemble des faces du support ou même appliquer plusieurs couches sans sortir du cadre l'invention.

Egalement, l'épaisseur du support de collagène peut varier dans de large limite. Elle sera, par exemple, comprise entre 2 et 25 mm, en particulier entre 10 et 20 mm.

D'autres buts, caractéristiques et avantages de l'invention apparaîtront clairement à partir de la description explicative qui va suivre faite en référence à plusieurs exemples de réalisation de l'invention donnés simplement à titre d'illustration et qui ne sauraient donc en aucune façon limiter la portée de l'invention. Dans les exemples, les pourcentages sont donnés en poids, sauf indication contraire.

L'invention est également décrite en référence aux dessins annexés rapportant des essais comparatifs. Ainsi, dans les dessins :

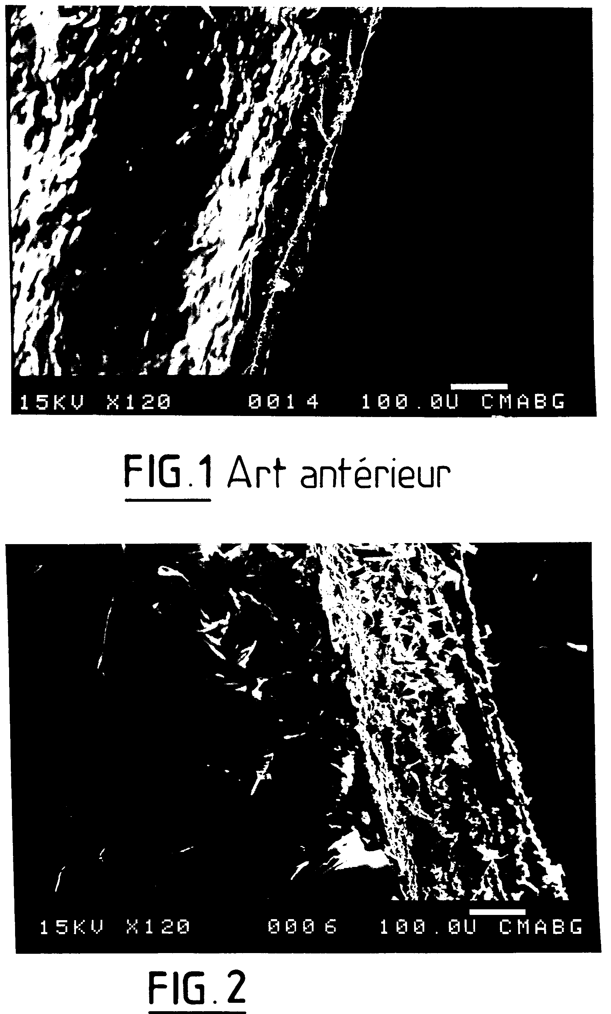

- la figure 1 représente une photo numérotée 0014 représentant la coupe d'un patch préparé selon la technique décrite dans le document FR-B1-2 628 634 selon la procédure décrite à l'exemple 4 permettant de mettre en évidence l'obtention d'une seule couche dans la structure du biomatériau qui apparaît homogène dans toute l'épaisseur, cette photo étant obtenue sur un microscope électronique fonctionnant à 15 kV et avec un agrandissement de 120 ; et
- la figure 2 représente une photo similaire numérotée 0006 obtenue par microscope électronique dans les mêmes conditions avec le même agrandissement, d'une membrane préparée selon l'invention faisant apparaître deux couches distinctes, la couche supérieure sur une face étant constituée de gélatine en étant révélée par la présence d'écailles caractéristiques.

Exemple 1

Préparation d'une membrane selon l'invention à propriété anti-adhérence à partir d'un collagène non réticulé ou non tanné, utilisable sous forme de pansement anti-adhérence

a) Préparation du gel de collagène natif essentiellement de type I et de faibles quantités de type III

Un gel de collagène natif de type I est préparé à partir de peaux de veaux préalablement lavées et épilées dans un bain contenant pour 100 g de peau: 250 ml d'eau, 2,5 g de sulfure de sodium à 60 % (environ 0,02 M en final), et 3,5 g de chaux éteinte (environ 0,15 M). Le bain d'épilage est éliminé par trois lavages de 250 ml d'eau.

La peau épilée est refendue afin d'éliminer les côtés chair puis déchaulée dans un bain contenant pour 100 g de peau : 50 ml d'eau, 3 g de chlorure d'ammonium (molarité finale environ 0,5 M), et 0,5 g de métabisulfite de sodium (0,02 M en final), elle est ensuite neutralisée, puis les sels sont éliminés par 2 lavages à l'eau.

Le derme ainsi traité est alors broyé, puis lavé dans de l'eau purifiée apyrogène à raison de 1 kg de broyat pour 5 l d'eau. Il est ensuite lavé par un tampon phosphate pH 7,8 : dihydrogénophosphate de potassium 0,78 g/l (molarité finale 0,005 M), et monohydrogénophosphate disodique 21,7 g/l (molarité finale 0,12 M). Le phosphate est ensuite éliminé par 2 lavages successifs à l'eau purifiée apyrogène.

Le broyat est alors acidifié par une solution d'acide acétique à 10 %, la quantité d'acide étant de 5 % p/p par rapport à la matière sèche, la molarité est d'environ 0,08 M en acide acétique. L'ensemble est alors malaxé, la pâte ainsi obtenue est diluée avec de l'eau purifiée apyrogène de façon à obtenir un gel ayant une concentration d'environ 0,75 % en collagène natif.

b) Préparation de la membrane proprement dite selon l'invention

Sur une couche préalablement congelée du gel à 0,75 % de collagène natif essentiellement de type I, obtenu à l'étape a) ci-dessus, d'un poids de 1 kg d'une épaisseur de 1,8 cm, sont coulés 214 ml d'une solution de gélatine qualité pharmacopée d'une concentration de 1,5 % en obtenant ainsi une couche continue de gélatine sur une face de support ayant une épaisseur de 4 mm. L'ensemble est ensuite lyophilisé, puis réticulé physiquement pendant 6 h à 110°C sous une pression de 450 µbar.

On obtient ainsi une membrane constituant une compresse qui peut être compactée en la pressant à chaud, par exemple à 55°C pendant 15 s sous une pression de 200 kg/cm$^2$.

La membrane compactée ainsi obtenue peut être utilisée telle quelle sous forme de pansement anti-adhérence comme cela sera décrit dans le cadre de l'exemple 3.

Exemple 2 selon l'invention

Membrane préparée à partir de collagène réticulé ou tanné utilisable comme pansement anti-adhérence

On prépare 1 kg de gel de collagène à 0,75 % de collagène essentiellement de type I comme décrit à l'exemple 1a).

Ce gel de collagène essentiellement de type I est ensuite coulé sur une épaisseur de 1,8 cm et lyophilisé.

On obtient ainsi une compresse qui est incubée pendant 24 h dans 375 ml de diméthylformamide (DMF) contenant 0,1 % (V/V) de diphénylphosphorylazide (DPPA). La compresse est ensuite rincée par deux passages dans 1 l de tampon borate à pH 8,9 contenant 15,256 g/l de tétraborate de sodium et 2,474 g/l d'acide borique. La compresse est finalement incubée dans un autre litre de même tampon pendant 12 h. Elle est alors rincée à l'eau permutée en continu pendant 6 h.

214 ml d'une solution de gélatine qualité pharmacopée à une concentration de 1,5 % sont alors coulés sur une face de la compresse préalablement congelée, en obtenant ainsi une couche de revêtement continue de gélatine, sur une face, d'une épaisseur de 4 mm. L'ensemble obtenu est ensuite lyophilisé.

On obtient alors une membrane selon la présente invention qui peut être utilisée telle quelle ou avantageusement peut être compactée en la comprimant à chaud, par exemple à 55°C pendant 15 s sous une pres-

6

sion de 200 kg/cm$^2$.

En variante, on peut couler une couche de gélatine sur les deux faces ou toutes les faces du support de collagène.

Exemple 3 selon l'invention

Effet anti-adhérence des membranes selon l'invention

La membrane selon l'invention tel que par exemple obtenue à l'exemple 1 peut être utilisée comme bio-matériau, en particulier en chirurgie, sous forme de pansement anti-adhérence.

Les qualités anti-adhérences des membranes selon l'invention ont été expérimentées in vivo chez le porc. Le protocole expérimental et les résultats obtenus sont donnés ci-dessous.

Huit porcs ont été opérés avec mise en place du produit entre l'organe opérant sur les tissus au moins avec la face ayant la couche de gélatine appliquée contre l'organe. pour éviter l'apparition des adhérences et sept porcs témoins ont subi les mêmes opérations chirurgicales. La race et le poids des animaux étaient les mêmes pour tous les sujets.

Trois membranes ont été implantées par animaux : une en position péricardique et deux contre le foie.

Les réinterventions ont eu lieu deux mois et demi plus tard. Le but de ce travail était d'analyser les aspects macro et microscopique des éventuelles adhérences post-opératoires et de tester la tolérance du produit après deux mois et demi d'implantation.

L'observation macroscopique des adhérences permet de les ranger dans quatre classes :

Classe 0 :     Absence totale d'adhérence

Classe I :     Adhérence minime, décollable au doigt

Classe II :    Adhérence modérée. La dissection est possible aux ciseaux et le plan de clivage est facile à repérer

Classe III :   Adhérence sévère. Pas de plan de clivage.

L'évaluation macroscopique s'appuie sur l'observation visuelle et sur des photos.

L'observation microscopique est destinée à la recherche de réaction inflammatoire éventuelle et à la mise en évidence d'apparition d'adhérence sur l'organe. L'observation a été réalisée d'une part lors de la résorption du produit et d'autre part après digestion totale du biomatériau.

Les résultats de l'expérimentation ont été les suivants : un décès s'est produit dans le groupe témoin et un autre après huit jours dans le groupe ayant reçu le produit. Le premier décès était dû à un accident d'anesthésie et le deuxième à une colite nécrosante hémorragique sans rapport avec l'utilisation du biomatériau. Cet incident a permis de procéder à une observation anatomique qui a montré qu'au bout de huit jours les pansements étaient indemnes de toute infection, bien fixés et non résorbés.

Les réinterventions effectuées au deuxième mois et demi ont permis de révéler que :

- les six animaux témoins présentaient tous des adhérences sévères de classe III,
- chez sept porcs du deuxième groupe, les adhérences étaient de classe 0 ou I. Dans certains cas, la résorption du biomatériau n'était pas complète. En particulier, le produit s'était transformé en un film adhérant au foie, permettant ainsi de décoller facilement cet organe du péritoine.

L'étude histologique a montré l'absence de réaction inflammatoire ainsi que la résorption plus ou moins complète du biomatériau.

Cette expérience met en évidence la grande efficacité (dans 100 % des cas sur 21 cas) de ce biomatériau et sa parfaite tolérance.

Exemple 4 selon l'invention

Essai comparatif de résistance mécanique, entre un patch de chirurgie viscérale obtenu en utilisant l'enseignement du document FR-B1-2 628 634 et une membrane à deux couches distinctes selon la présente invention.

Les conditions de préparation et d'essai de résistance mécanique sont les suivantes :

1°. Préparation d'un patch de chirurgie viscérale sur la base de l'enseignement de FR-B1-2 628 634

A 1 kg de gel de collagène natif bovin de type I et de type III obtenu selon le procédé décrit dans la présente invention à l'exemple 1a, on ajoute 5 l d'eau purifiée à pyrogène. Le nouveau gel obtenu présente une proportion de matière sèche de 0,125 %.

Le collagène de cette préparation est précipité à l'aide de 660 ml de solution $Na_2HPO_4$ (0,2 M, pH 7,5). La précipitation est réalisée à 20°C pendant 15 h.

Avec le précipité récupéré par centrifugation, on réalise un gel contenant 0,76 % de collagène et 0,76 % de glycérine comme décrit à l'exemple 1 de FR-B1-2 628 634 avant d'être congelé et lyophilisé.

7

Pour réaliser cet essai comparatif, l'éponge obtenue est alors pressée à 55° pendant 15 s sous une pression de 200 kg/cm² comme préconisé dans le cadre du procédé de la présente invention, les conditions de compression n'étaient pas décrites dans ce document antérieur.

Ensuite, 164 ml d'une solution de gélatine à une concentration de 1,5 % en poids sont coulés sur une face de l'éponge comprimée, ce qui forme une couche continue de 4 mm sur cette face. L'ensemble est alors séché à l'air à température ambiante.

Le patch de chirurgie viscérale ainsi obtenu présente une épaisseur d'environ 600 µm (0,6 mm).

Sur une partie de ce patch, on réalise une coupe et une prise de photographie sur microscope électronique pour obtenir la photo de la figure 1.

On prépare par ailleurs 10 éprouvettes à partir de ce patch ayant une largeur de 15 mm et une épaisseur de 600 µm. On fait subir une hydratation de 1 min dans de l'eau distillée.

Les résultats obtenus avec le patch de chirurgie viscérale selon l'art antérieur sont rapportés au tableau I.

## TABLEAU I

### Détermination de la résistance à la traction avec allongement à la rupture d'un patch selon FR–B1–2 628 634

| | Résistance à la traction en N | Allongement à la rupture en % |
|---|---|---|
| Eprouvettes | | |
| Essai 1 | 0,48 | 30 |
| Essai 2 | 0,79 | 33 |
| Essai 3 | 0,53 | 32 |
| Essai 4 | 0,56 | 26 |
| Essai 5 | 0,102 | 34 |
| Essai 6 | 0,102 | 40 |
| Essai 7 | 0,48 | 24 |
| Essai 8 | 0,102 | 34 |
| Essai 9 | 0,52 | 24 |
| Essai 10 | 0,80 | 30 |
| Moyennes | 0,45 ± 0,26 | 30,7 ± 5,03 |

2°. Préparation d'une membrane selon l'invention

La membrane utilisée est celle qui est obtenue à l'exemple 1. On prépare 10 éprouvettes ayant une largeur de 15 mm et une épaisseur de 600 µm (0,6 mm) pour être identique aux éprouvettes utilisées pour réaliser les essais de résistance mécanique du patch selon FR-B1-2 628 634.

On réalise également une photo en microscopie électronique dans les mêmes conditions que pour l'art antérieur, objet de la figure 2.

Les résultats des essais de résistance à la traction avec allongement à la rupture des éprouvettes selon la présente invention sont rapportés au tableau II.

## TABLEAU II

Détermination de la résistance à la traction avec allongement à la rupture
d'une membrane selon l'exemple 1 de la présente invention

| Eprouvettes | Résistance à la traction en N | Allongement à la rupture en % |
|---|---|---|
| Essai 1 | 3,53 | 34 |
| Essai 2 | 1,35 | 30 |
| Essai 3 | 1,85 | 14 |
| Essai 4 | 3,75 | 28 |
| Essai 5 | 3,37 | 34 |
| Essai 6 | 2,20 | 22 |
| Essai 7 | 1,90 | 16 |
| Essai 8 | 3,57 | 32 |
| Essai 9 | 3,35 | 28 |
| Essai 10 | 2,75 | 17 |
| Moyennes | 2,76 ± 0,87 | 25,5 ± 7,65 |

Il est à noter que les essais de résistance à la traction ont tous été réalisés avec un appareil de marque Instron type 6022 bien connu à l'homme de l'art.

Il résulte clairement de la comparaison des tableaux I et II précités que la résistance à la traction en Newton moyenne des éprouvettes de membrane selon la présente invention est de 2,76 ± 0,87 alors que celle-ci n'est que de 0,45 ± 0,26 dans le cadre du patch selon FR-B1-2 628 634 ce qui constitue une amélioration remarquable de résistance mécanique d'environ 500 %.

On comprend ainsi le progrès technique considérable obtenu grâce à l'invention.

## Revendications

1. Membrane à base de collagène, caractérisée en ce qu'elle comprend un support à base dudit collagène revêtu complètement sur au moins une face d'une couche de gélatine, la couche de gélatine étant essentiellement non mélangée au support de collagène, de préférence l'ensemble de la couche de gélatine et du support à base de collagène étant à l'état lyophilisé.

2. Membrane selon la revendication 1, caractérisée en qu'elle se présente sous forme compacte, en particulier en ayant été comprimée à chaud.

3. Membrane selon la revendication 1 ou 2, caractérisée en ce que la membrane est obtenue en déposant une solution de gélatine sur un gel de collagène préalablement congelé, avant lyophilisation de l'ensemble.

4. Membrane selon la revendication 1 ou 2, caractérisée en ce que ladite membrane est obtenue en préparant tout d'abord une éponge de collagène par lyophilisation d'un gel de collagène, ensuite une étape de réticulation du collagène réalisée par un agent réticulant qui peut être, par exemple, choisi parmi le diphénylphosphorylazide (DPPA) en obtenant ainsi après lyophilisation et réticulation, une éponge réticulée qui est réhydratée puis congelée, avant de couler une solution de gélatine pour couvrir complètement au moins une face du support de collagène d'une couche de revêtement continue de gélatine.

5. Membrane selon la revendication 4, caractérisée en ce que l'ensemble obtenu est lyophilisé.

6. Membrane selon la revendication 5, caractérisée en ce que le lyophilisat obtenu est comprimé à chaud pour obtenir une membrane comprimée.

7. Membrane selon l'une des revendications 1 à 6, caractérisée en ce que le collagène est essentiellement de type I, et de préférence provient de la peau de veau.

8. Membrane selon l'une des revendications précédentes, caractérisée ce que le collagène précité est essentiellement de type I, sous forme complètement native, c'est-à-dire avec sa structure hélicoïdale et ses télopeptides préservés.

9. Membrane selon l'une des revendications précédentes, caractérisée en ce que le support précité est à base de collagène essentiellement de type I se présentant sous forme d'une nappe obtenue par lyophilisation d'un gel de collagène essentiellement de type I.

10. Membrane selon l'une quelconque des revendications 1 à 3, 7 à 9, caractérisée en ce que le support de collagène a subi une réticulation physique, en particulier par séchage sous vide à une température au moins égale à 80°C, et de préférence au moins égale à 100°C.

11. Membrane selon l'une quelconque des revendications précédentes, caractérisée en ce que la membrane obtenue après lyophilisation a été comprimée à chaud sous une pression élevée, par exemple supérieure à 100 kg/cm$^2$ et en particulier de l'ordre de 200 kg / cm$^2$.

12. Procédé de fabrication d'une membrane à base de collagène, caractérisé en ce qu'il comprend les étapes essentielles suivantes :
a) on prépare tout d'abord un gel de collagène, de préférence un collagène de type I ;
b) on traite le gel de collagène dans des conditions rendant le collagène non fluide et non capable d'être miscible à une solution ;
c) on prépare une solution de gélatine et on coule cette solution de gélatine sur au moins une face du collagène traité pour être non miscible avec ladite solution de gélatine ; et
d) on sèche, de préférence par lyophilisation, l'ensemble formé par la couche de gélatine recouvrant complètement au moins ladite face de collagène, en obtenant ainsi ladite membrane.

13. Procédé selon la revendication 12, caractérisé en ce que, après lyophilisation, on réalise une réticulation physique de la membrane, en particulier en soumettant la membrane à un traitement sous pression réduite à la chaleur, en particulier à une température supérieure à 80°C et mieux supérieure à 100°C.

14. Procédé selon la revendiction 13, caractérisé en ce que la pression réduite précitée est inférieure à 1 mbar et de préférence inférieure à 0,5 mbar.

15. Procédé selon l'une des revendications 12 à 14, caractérisé en ce que le traitement du gel de collagène pour le rendre non fluide et non miscible comprend une étape de congélation.

16. Procédé selon la revendication 15, caractérisé en ce que le traitement du gel de collagène comprenant une lyophilisation, puis une réhydratation suivie d'une congélation avant de couler la solution de gélatine.

17. Procédé selon la revendication 16, caractérisé en ce que le gel de collagène lyophilisé, constituant une éponge de collagène, est soumis à une étape de réticulation du collagène par un agent réticulant qui peut être, par exemple, choisi parmi le diphénylphosphorylazide (DPPA) en obtenant ainsi une éponge lyophilisée, réticulée, qui est ensuite réhydratée et congelée avant de couler la solution de gélatine précitée sur au moins une face.

18. Procédé selon l'une des revendications 12 à 17, caractérisé en ce que la membrane obtenue après lyophilisation de l'ensemble de la solution de gélatine et du collagène est compactée, en particulier compactée à chaud, à une pression avantageusement au moins égale à 100 kg/cm$^2$, en particulier d'environ 200 kg/cm$^2$.

19. Procédé selon l'une des revendications 12 à 18, caractérisé en ce que dans le cas où la membrane est utilisée comme biomatériau, en particulier en chirurgie, dans des points de l'organisme où la dégradation enzymatique sera importante, on utilise une membrane préparée à partir d'un gel de collagène qui aura été tout d'abord lyophilisée pour former une éponge, qui est ensuite soumise à un traitement de réticulation ou de tannage, en particulier par le diphénylphosphorylazide, puis réhydratée et congelée avant d'être enduite ou recouverte de la solution de gélatine sur au moins une face, puis l'ensemble sera à nouveau lyophilisé.

20. Procédé selon la revendication 19, caractérisé en ce que le lyophilisat obtenu est ensuite comprimé à chaud pour obtenir la membrane sous sa forme finale directement utilisable comme biomatériau de chirurgie.

21. Utilisation de la membrane telle que définie dans l'une quelconque des revendications 1 à 11, ou telle qu'obtenue par le procédé selon l'une quelconque des revendications 12 à 20, comme biomatériau, en particulier en chirurgie et notamment sous forme de pansement anti-adhérences.

22. Pansement anti-adhérences, caractérisé en ce qu'il comprend au moins une membrane telle que définie à l'une quelconque des revendications 1 à 11 ou tel qu'obtenu par le procédé selon l'une quelconque des revendications 12 à 20.

23. Pansement anti-adhérences selon la revendication 22, caractérisé en ce qu'il s'agit d'un pansement anti-adhérences en chirurgie abdominale, en gynécologie ou en chirurgie cardiaque.

FIG.1 Art antérieur

FIG.2

EP 0 686 402 A1

**Office européen des brevets**

**RAPPORT DE RECHERCHE EUROPEENNE**

Numero de la demande

EP 95 40 1185

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | WO-A-89 08467 (IMEDEX) <br> * le document en entier * <br> --- | 1 | A61L31/00 |
| A | EP-A-0 213 563 (JOHNSON & JOHNSON) <br> * page 9, ligne 20 * <br> ----- | 1 | |

**DOMAINES TECHNIQUES RECHERCHES (Int.Cl.6)**

A61L

Le présent rapport a été établi pour toutes les revendications

| Lieu de la recherche | Date d'achèvement de la recherche | Examinateur |
|---|---|---|
| LA HAYE | 30 Juin 1995 | Peltre, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P04C02)

13